# EUROPÄISCHE PATENTSCHRIFT

(11) **EP 2 865 651 B1**
(45) Veröffentlichungstag und Bekanntmachung des Hinweises auf die Patenterteilung: **22.06.2016**
(21) Anmeldenummer: 15000211.1
(22) Anmeldetag: 17.03.2012
(51) Int. Cl.: C02F 1/44, A61L 2/04, A61M 1/16, B01D 63/10, B01D 61/02, B01D 61/12, B01D 61/58, B01D 65/02, C02F 1/48, C02F 1/467, C02F 1/38, C02F 1/02, B01D 61/24, C02F 1/78, C02F 103/02

(54) **RO-(UMKEHROSMOSE) ANLAGE**
REVERSE OSMOSIS ASSEMBLY
INSTALLATION RO (OSMOSE INVERSÉE)

(30) Priorität: 27.05.2011 DE 102011102662
(43) Veröffentlichungstag der Anmeldung: 29.04.2015
(62) Teilanmeldung aus: 12001827.0
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- DE-A1- 10 262 036
- DE-C1- 3 941 131
- JP-A- 2 035 916
- JP-A- 2 227 128
- Thomas Melin ET AL: "Modulkonstruktionen. Module mit Flachmembranen. Wickelmodul" In: "Membranverfahren. Grundlagen der Modul- und Anlagenauslegung", 31. Dezember 2007 (2007-12-31), Springer Verlag, XP55170968, ISBN: 978-3-54-034327-1 Seiten 173-175, * Seiten 173-175 *

## Beschreibung

Die Erfindung betrifft eine Vorrichtung zur Wasseraufbereitung nach dem Prinzip der umgekehrten Osmose. Vorrichtungen dieser Art, Umkehrosmose- oder RO-Anlagen, werden insbesondere in Verbindung mit Hämodialysegeräten eingesetzt, um aus Leitungswasser hochreines, keimfreies Wasser zur Bereitung der Dialysierflüssigkeit zu gewinnen.

Die Erfindung zielt insbesondere darauf ab, möglichst keimfreie Flüssigkeit zu erreichen.

Diese Aufgabe wird durch die Merkmale des Anspruchs 1 gelöst. Weitere Einzelheiten und Ausgestaltungen der Erfindung ergeben sich aus den Unteransprüchen und der nachfolgenden Beschreibung von Ausführungs-beispielen in Verbindung mit den Abbildungen.

Dabei zeigen:
- Fig. 1: das Schema einer typischen Umkehrosmoseanlage nach dem Stand der Technik,
- Fig.2: das Schema einer vergleichbaren Umkehrosmoseanlage mit Ausstattungsmerkmalen entsprechend der Erfindung,
- Fig. 2a: eine alternative Ausführungsform,
- Fig.3 bis 4: das Schema entsprechender Vorrichtungen.

Das Funktionsprinzip von Umkehrosmoseanlagen besteht bekanntlich darin, dass das aufzubereitende Wasser in einem Filtermodul unter hohem Druck an der Oberfläche einer semipermeablen Membran entlanggeführt wird, wobei ein Teil des Wassers, das sogenannte Permeat, so über die Oberfläche der spiralförmig gewickelten Membran geführt wird, dass es durch die Membran tritt und auf der anderen Seite der Membran innerhalb des Moduls in einem Permeatsammelrohr gesammelt und von dort über hydraulische Verbindungen den Verbrauchsstellen zugeführt wird.

Der nicht durch die Membran tretende, mit zurückgehaltenen Stoffen angereicherte Teil des Rohwassers, das sogenannte Konzentrat, fließt am Ende der Strömungsstrecke aus dem Filtermodul aus.

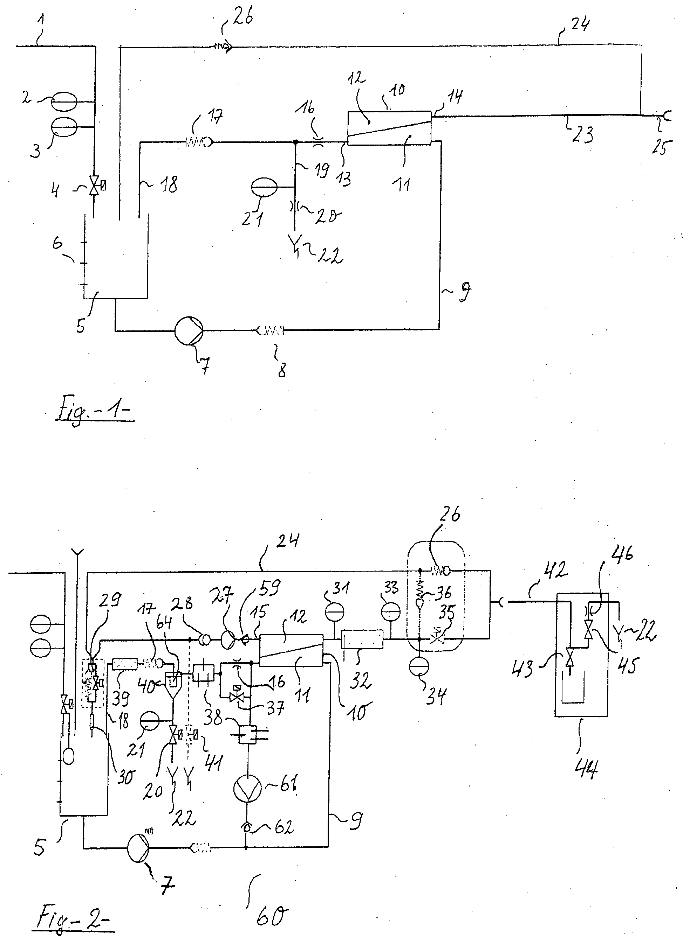

Das in Fig. 1 gezeigte Schema veranschaulicht als typisches Beispiel das Zusammenwirken wesentlicher Funktionselemente einer Umkehrosmoseanlage nach dem Stand der Technik. Das aufzubereitende Rohwasser fließt aus der zuführenden Leitung 1 und über das Ventil 4 in einen Vorlaufbehälter 5 mit eingebauter Füllstandsregelung 6. Aus diesem Vorlaufbehälter 5 gelangt das Wasser durch die Leitung 9 über die Pumpe 7 in den Filtermodul 10, dessen Primärraum 11 durch die semipermeable Membran 49 von dem Sekundärraum 12 getrennt ist. Aus dem Sekundärraum 12 fließt das Permeat in eine Ringleitung 23/24 von der die Verbraucherleitungen 25 abzweigt. Überschüssig erzeugtes Permeat kann am Ende der Ringleitung 23/24 über ein eingefügtes Druckhalteventil 26 in das Gefäß 5 zurückfließen, wobei die Einstellung dieses Ventils den in der Ringleitung 23/24 herrschenden Druck bestimmt.

Der für die Filtration notwendige Druck im Primärraum 11 des Filtermoduls10 wird durch die Pumpe 7 in Verbindung mit einem in die Konzentratleitung 13 stromabwärts vom Filter eingefügten Strömungswiderstand 16, z.B. in Form eines Drossel- oder eines Druckventils, erzeugt.

Umkehrosmosen dienen insbesondere auch der Gewinnung von keimfreiem Wasser.

Der nicht durch die Filtermembran 49 tretende, mit zurückgehaltenen chemischen Wasserinhaltsstoffen und Bakterien angereicherte Teil des zugeführten Leitungswassers bildet einen Biofilm auf den Innenflächen des flüssigkeitsführenden Systems.

Die Ausscheidungen des Biofilms im Primärraum 11 können als Pyrogene und Endotoxine die nicht ideale Filtermembran 49 passieren und kontaminieren die hochreine Permeatseite 12/23/24.

Dort lagern sich diese Teile als Biofilm ab und kontaminieren das Permeat.

Zur Vereinfachung wird nachfolgend zur Beschreibung der Dekontaminations-Desinfektions- und Reinigungsmaßnahmen der Begriff Reinigung gewählt.

Ein Grund für die Keimanreicherung des Permeat liegt auch in dem nicht und nur mäßige durchflossenen Bereichs des Permeatsammelrohres. Dieses Leitungsstück der hochreinen Sekundärseite ist nur mit größerem Aufwand thermisch oder chemisch zu desinfizieren bzw. zu reinigen.

Konstruktiv ist die Filtermembran des Umkehrosmosemoduls spiralförmig um ein Permeatsammelrohr gewickelt. Dabei wird ein Teil der Flüssigkeit von der Primär- zur Sekundärseite durch die Membrane hindurch zum Permeatsammelrohr geführt und von dort dem Verbraucher zugeführt.

Die Ursache der Keimanreicherung im Permeatsammelrohr ist zum einen der eingeschränkte, relativ geringe transmembrane Fluss durch die Filtermembran zum Permeatsammelrohr und zum anderen setzt die Reinigung des Permeatsammelrohres immer eine transmembrane Reinigung - also eine Reinigung der Primärseite - und damit des Gesamtsystems- voraus und diese ist kosten- und zeitintensiv.

Aber auch bei einer Reinigung des sekundärseitigen Verteilungssystems besteht die Problematik, dass keine garantierte Keimfreiheit, insbesondere auf der Grundlage normativer Vorgaben gewährt werden kann, weil eine Rückverkeimung aus dem Permeatsammelrohr heraus sehr wahrscheinlich ist.

Ein anderer Grund für Rückverkeimungen ist, dass bei den bekannten Anwendungen, das Keimwachstum innerhalb des sekundärseitigen Verteilungssystems nur bis zum Übergabepunkt am Verbraucher mittels Reinigungsmaßnahmen verhindert wird.

Z.B wird bei Dialysegeräten mit einem freien Einlauf gemäß EN 1717 die Strecke vom Übergabepunkt der Reinstwasserleitung der Umkehrosmose bis zum freien Einlauf des Dialysegerätes nicht berücksichtigt.

Auch bei der Bevorratung von Permeat z. B. bei Reinstwassertanks bzw. Beuteln wird die Füllleitung nicht vollständig in den Desinfektionsvorgang mit einbezogen. Diese Problematik gilt auch für Mischanlagen zur Herstellung medizinischer Lösungen zum einen aus pulvrigen, pastösen, granulierten oder anderen hochkonzentrierten Rohstoffen und zum anderen aus hochreinem Permeat, sofern diese Anlagen einen Permeatspeicher mit Zuführleitungen für das Permeat oder die Permeaterzeugung beinhalten.

Um diese Leitungsstrecke keimfrei zu halten, wären erhebliche Ressourcen in Form von elektrischer Energie bei einer Heissreinigung oder auch von chemischen Desinfektionsmitteln, wie auch der eingesetzten .Personalkapazität notwendig, weil in der Regel nicht nur das sekundäre Verteilungssystem, sondern auch die RO-Anlage und der angeschlossene Verbraucher z.B. das Dialysegerät in den Desinfektionsvorgang mit eingebunden werden müssen.

Dabei birgt insbesondere der Eintrag von großen Mengen an chemischen Desinfektionsmitteln neben der Kontamination der Abwässer auch erhebliche Sicherheitsrisiken für den Patienten, weil geringste Rückstände im Gerät oder in den Leitungen toxische Auswirkungen auf den Patienten haben. Diesen kann nur mit großen Ausspülvolumen und einer sorgfältigen Kontrolle auf Rückstände vorgebeugt werden.

Üblicherweise werden deshalb die Desinfektionsmaßnahmen auf die jeweiligen angeschlossenen Geräte begrenzt und die Schnittstellen bzw. die Reinstwasserübergabestellen nicht berücksichtigt.

Ausnahmen sind dabei integrierte Heissreinigungen bei denen große Mengen an heißem Wasser aufbereitet und z.B. Dialysegeräten zugeführt und durch diese hindurch zum Abfluss befördert werden.

Angestrebt ist es, eine garantierte Keimfreiheit des hochreinen Verteilerkreises inkl. der angeschlossenen Verbraucherleitung zuzusichern und trotzdem die Betriebskosten für Hygienemaßnahmen zu reduzieren.

Notwendig ist eine vollautomatische, für Patient und Anwender risikofreie Reinigung, ohne dass dabei wie z.Zt. überflüssigerweise durchgeführt, die Umkehrosmose und der angeschlossenen Verbraucher zwingend mit Chemie zu desinfizieren sind.

Dabei soll niedrigster Energieeintrag und keine negative Beeinflussung der Abwässer durch Chemie oder thermische Energie das Ziel sein.

Eine partielle Desinfektion des gesamten hochreinen Verteilungssystems also der Sekundärseite, auch innerhalb des angeschlossenen Verbrauchers bei Dialysegeräten bis zum Wassereingang, also den Vorlaufbehälter, ist anzustreben. Dabei können unterschiedliche Reinigungsverfahren auf Primär und Sekundärseite der Membran durchführbar sein. Wobei eine komplette integrierte Reinigung des Gesamtsystems auch möglich ist.

Es ist eine Messmöglichkeit zum Ausmaß der Verunreinigung /Verkeimungsgrades und einer daraus resultierenden Warnung bzw. der automatischen Einleitung einer Reinigung erforderlich.

Eine Kontrolle auf Desinfektionsmittelfreiheit soll nicht erforderlich sein.

Es sind schonende Reinigungsverfahren zu wählen um eine Lebensdauereinschränkung der eingesetzten Komponenten, insbesondere der FilterMembran zu verhindern.

Die DE 102 62 036 A1 offenbart eine Reinstwasserversorgungsanlage mit einem Permeat-Zirkulationskreis, in den Permeat aus dem Sekundärraum eines Filtermoduls 15 durch eine Zulaufleitung fließt. Dabei durchströmt das Permeat ein in der Zulaufleitung angeordnetes Permeatrückschlagventil, das verhindert, dass Permeat in den Sekundärraum des Filtermoduls zurück strömt. Der Zirkulationskreis kann gereinigt und von schädlichen Substanzen befreit werden, die durch ein Ventil in ein Puffergefäß ausgespült werden können. Dabei werden aber nicht der Sekundärraum des Filtermoduls und die zu dem Zirkulationskreis führende Stichleitung in den Reinigungsvorgang einbezogen. Wenn das Permeat beim Reinigungsvorgang durch das geöffnete Ventil in das Puffergefäß und von dort in den Filter strömt, gelangt es durch die Membran in den Sekundärraum. Wenn der Sekundärraum durch ein Permeatsammelrohr gebildet ist, wird dabei ein Endbereich des Permeatsammelrohres nicht wirkungsvoll durchströmt, so dass sich dort schädliche Ablagerungen anlagern können.

Bei der aus der DE 3941131 C1 bekannten Umkehrosmoseanlage geht ebenfalls von dem Sekundärraum des Filtermoduls nur eine Permeatleitung ab.

Die Erfindung sieht vor, dass der Sekundärraum des Filtermoduls durch ein innerhalb von spiralförmig angeordneten Membrantaschen angeordnetes, perforiertes Permeatsammelrohr gebildet ist, das über Anschlüsse an beiden Enden mit dem Zirkulationskreis verbunden ist, indem es mit seinem anderen Ende mit der Permeat-Rücklaufleitung so verbunden ist, dass der Zirkulationskreis gebildet ist, in dem das Permeat zirkulieren kann.

Dabei wird das Filtermodul der Umkehrosmoseanlage als 4 Pol betrieben und es entsteht ein geschlossener, unabhängig vom Primärkreis der Umkehrosmoseanlage zu reinigender Sekundär-Kreislauf mit minimalsten Toträumen.

Gemäß der Erfindung wird dafür im Sekundärkreislauf eine weitere Pumpe eingesetzt. Dadurch sind Primär- und Sekundärseite unabhängig voneinander mittels unterschiedlichen Reinigungsverfahren und Intervallen zu reinigen.

Die Erfindung sieht mit Vorteil ein Reinigungsverfahren vor bei dem eine Ozonisierung des sekundären Verteilungssystem stattfindet. Dies kann auch im Wechsel mit einer Heißreinigung der Umkehrosmose oder auch des Gesamtsystems stattfinden. Wobei eine chemische Reinigung oder eine Kombination aus thermischer und chemischer Reinigung ausdrücklich nicht ausgeschlossen ist.

Es wurde auch festgestellt, dass die Kombination von niedrigst dosiertem Ozon und erhöhter Temperatur im Wasser nur minimalste Materialschäden bewirken, weil Oxidations- und Zerfallsprozess des Ozons beschleunigt werden. Sodass mit dieser Methode auch eine Ozonisierung der Umkehrosmose Primärseite und damit der Membran ermöglicht wird.

Ebenso ist ein Ozoneintrag in einen evtl. angeschlossenen Bevorratungsbehälter z.B. bei einer mit Umkehrosmoseanlage in Verbindung stehenden Mischanlage sinnvoll um die mit Permeat angesetzte Lösung mikrobiologisch stabil zu halten.

Mit großem Vorteil wird neben dem Wassereingangsventil des Verbrauchers insbesondere bei Dialysegeräten ein weiteres Ventil zum Abfluss geschaltet. Dieses

Ventil leitet während einer Reinigung das Reinigungsmittel bis zum Eingangsventil des angeschlossenen Verbrauchers. Dadurch kann ohne Betrieb des Verbrauchers die Schnittstelle vom Verteilungssystem der Umkehrosmose bis zum Dialysegeräte Wassereingang nahezu totraumfrei gereinigt werden.

Die Erfindung sieht mit Vorteil eine Reinigungskammer im Primärzirkulationskreis der Umkehrosmose vor, deren Konstruktion eine elektrische oder magnetische oder elektromagnetische oder elektrolytische oder sonographische oder eine Kombination verschiedener physikalischer Einwirkungen der durchfließenden Flüssigkeit zulässt und vorsieht.

Die Aufgabe der Reinigungskammer ist zum einen die Dekontamination der Mikroorganismen u. zum anderen die Stabilisierung der Härtebildner, so dass Ablagerungen auf der Umkehrosmosemembran verhindert werden.

Die Anwendung und der Einbauort der Reinigungskammer sind jedoch nicht auf die beschriebene Funktion beschränkt.

Da die Desinfektionswirkung der elektrolytisch erzeugten Sauerstoffradikale als auch die Stabilisierung der Kalkkristalle in der Flüssigkeit nach dem Abschalten der Reinigungskammer nur temporär vorhanden sind, wird vorteilhaft periodisch und oder am Ende eines Betriebszyklus die Hochdruckdrossel entweder motorisch, oder falls ein fixer Strömungswiderstand eingebaut ist, mittels Bypassventil mit Abflussventil geöffnet. Dadurch wird die Strömung im Primärzirkulationskreis schlagartig erhöht und die Oberflächen der Flüssigkeit führenden Komponenten schwallartig überströmt und ausgespült.

Da die Wirkung der Reinigungskammer durch ihren physikalischen Effekt oder ihre Effekte auf die Kristallbildung durch den Anwender nicht unmittelbar feststellbar ist, wird mit großem Vorteil für den Primärraum ein Reinigungssensor vorgesehen.

Dabei können Komponenten oder flüssigkeitsführende Leitungen mit transparentem oder transluzentem Material ausgestaltet sein, um visuell oder opto- elektronisch die Verunreinigung zu überprüfen.

Bei einer vorteilhaften Ausgestaltung ist die zugehörige Sender-Empfängereinheit auf einer Ebene angeordnet. Das opt. Sendersignal wird dabei auf einer gegenüberliegenden Spiegelfläche projektiert und von dort zum opt. Empfänger zurückgestrahlt.

Die bevorzugte Lösung ist ein transparentes Rohrstück mit gegenüberliegenden Sender- Empfängersensoren. Die Quantität des Empfängersignals ist dabei eine direkte Funktion des Verschmutzungsgrades.

Mit großem Vorteil kann zur Verbesserung der zeitlichen Einwirkung und Verstärkung der physikalischen Reinigungseffekte eine zusätzliche Zirkulations-pumpe mit einer Reinigungskammer zwischen dem Konzentratausgang und dem Mischwassereingang angeschlossen werden. Dabei kann es sich um eine zusätzliche Reinigungskammer mit einem differenten physikalischen Effekt zur Reinigungskammer handeln.

Die Durchströmung des Primärraumes im Sinne einer optimalen Überströmung der Membran 49 ist dabei gewährleistet, und zwar weitgehend unabhängig von der Tätigkeit der für die Mischwasser-Zuführung, den Druckaufbau und Zirkulationsleistung der eingesetzten Pumpe 6.

Um Stoffrückstände zu entfernen besteht ein weiteres Erfindungsmerkmal darin, dass die Flüssigkeit des Primärzirkulationskreises mittels tangentialen Einlauf durch eine zylindrische Zentrifugalkammer geleitet wird, an deren oberen Ende eine durch Flüssigkeitsdruck drehbare Turbinenschaufel die zu trennenden Stoffteilchen und Partikel nach unten fördert und die gereinigte Flüssigkeit durch eine Hohlwelle bzw. einen siebförmigen Zylinder nach oben weiterleitet.

Unterhalb der Zentrifugalkammer befindet sich ein Sammelraum für die abzuscheidenden Stoffteilchen bzw. Kalkcluster. Bei vorhandener Zentrifugalkammer kann das Abflussventil 26 am Sammelraum befestigt sein. Wobei die Zentrifugenkammer auch stromaufwärts des Primärkreises z.B. vor dem Vorlaufbehälter (5) angeordnet sein kann.

Mit großem Vorteil wird auch vorgeschlagen die Leistungsendstufen für die Ansteuerung der Reinigungskammer hinsichtlich Frequenz und Strom einstellbar zu machen und durch den Prozessor der Umkehrosmoseanlage anzusteuern und auf Störung zu überwachen. Dabei kann ein bestimmtes Bitmuster als Testsignal ausgegeben und mittels *watch dog* überwacht werden. Wobei der jeweilige Betriebsstatus wie auch der Signalverlauf des über die Anzeigevorrichtung der Umkehrosmoseanlage angezeigt und mit Speicherbausteinen abgespeichert werden. Mittels Schnittstelle z.B. Ethernetanschluss des Mikrocontrollers der Umkehrosmoseanlage sind die Daten jederzeit abrufbar.

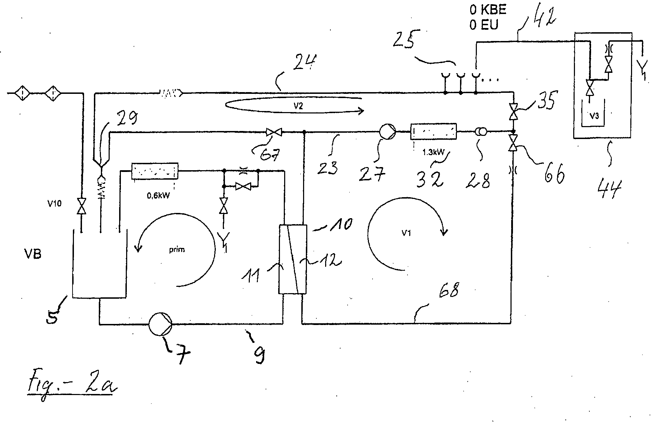

Figur 2 zeigt das Modul (10) mit dem Mischwasserzufluss (9), dem Konzentratabfluss (13), der Permeatzuführung (14) und der Permeatzirkulationsleitung (15). Dabei wird der Membranprimärraum (11) über die Pumpe (7), die Hochdruckdrossel (16), die Reinigungskammer (38) und die Zentrifugalkammer (40) über das Druckhalteventil (17) und die Heizung (39) so betrieben, dass ein ausreichend hoher Transmembrandruck entsteht, um die Flüssigkeit des Primärraumes (11) in den Membransekundärraum (12) zu befördern. Bedingt durch den hohen Transmembrandruck wird die Flüssigkeit des Membransekundärraums (12) (Permeat) über die Temperatursensoren (31 / 33), die Heizung (32), die Leitfähigkeitszelle (34) und das Ventil (35) über die Leitung (42) zum Verbraucher (44) geführt. Dabei ist die Pumpe (27) wahlweise zu betreiben.

Bei nichtangeschlossenem Verbraucher (44) kann die Flüssigkeit entweder über die Leitungen (23 / 24) oder, bei geschlossenem Ventil, über das Permeatringsicherheitsventil (36) zum Vorlaufbehälter (5) zurückgeführt werden.

Zur Reinigung des hochreinen Sekundärkreislaufs (12/23/24/48) zirkuliert die Pumpe (27) bei einer thermischen Reinigung die Flüssigkeit über den Heizer (32), bis eine mikrobiologisch inaktivierende Temperatur erreicht ist.

Bei Reinigung des Sekundärkreislaufs mittels elektrolytischer Ozonzelle (28) wird diese über eine Sicherheitsschaltung zugeschaltet und das Ozon-Flüssigkeitsgemisch mittels Pumpe (27) so lange im Kreislauf zirkuliert, bis eine mikrobiologisch inaktivierende Wirkung erzielt wurde.

Dabei kann der Primärkreislauf (11/9/18) mit der Pumpe (7) deaktiviert werden, so dass ausschließlich eine Reinigung des hochreinen Kreislaufs (12/23/24) erfolgt.

Lage und Betriebsart der Pumpe (27), sowie der Ozonzelle (28) sind nicht auf das beschriebene Verfahren beschränkt.

Erfolgt eine integrierte Desinfektion d.h.mit angeschlossenem und betriebenen Verbraucher (44), kann entweder über das geöffnete Ventil (43) der Verbraucher mit Heißwasser oder Ozon versorgt werden. Ebenso ist eine Desinfektion der Leitung (42) über das geöffnete Ventil (45) ohne Betrieb des Verbrauchers (44) zur Drainage (22) möglich. Das Drosselventil (46) reduziert dabei den Volumenstrom. Die Schaltung der Verbraucherventile (43 / 45) bzw. die Kommunikation zwischen Verbraucher (44) und RO-Anlage (60) erfolgt über Kommunikationsschnittstellen der RO (60) bzw. des Verbrauchers (44). Dabei können die Kommandos sowohl von der RO (60) an den Verbraucher (44) als auch umgekehrt erfolgen.

Zur Heißreinigung der gesamten RO-Anlage (60) ohne Verbraucher (44) wird das Ventil (29) geöffnet. Dabei versorgt die Pumpe (7) den Flüssigkeitskreislauf so, dass ausreichend Permeat über die Heizung (32) fließt. Ventil (37) kann dabei geöffnet werden. Die Pumpe (27) zirkuliert unterstützend im Sekundärkreislauf (23/24/48). Das aufgeheizte Permeat fließt wahlweise über die Ventile (26 / 35) oder (36) zum Vorlaufbehälter (5) zurück, bis ein mikrobiologisch inaktivierender Status erreicht ist.

Es ist auch möglich, Ozon in geringen Mengen in den Primärkreislauf (9/18) einzutragen, um dort eine desinfizierende Wirkung ohne Oxidation des Membranmaterials zu erreichen. In Kombination mit dem Ozoneintrag kann ein Betrieb der Heizung (32) so erfolgen, dass lediglich eine Material schonende Kurzzeitozonisierung durchgeführt wird.

Neben der Heißreinigung der gesamten Umkehrosmoseanlage besteht die Möglichkeit mittels Reinigungskammer (38) die Flüssigkeit des Primärkreislaufs permanent zu reinigen. Dazu wird die Flüssigkeit bei laufender Pumpe (7) über die Reinigungskammer (38) und Zentrifugalkammer (40) geführt. Ein Teil der zirkulierenden Flüssigkeit wird über den Konzentratflussmesser (21) und das Konzentratabflussventil (20) zum Ausgang geführt.

Zum Ausspülen von Stoffrückständen wird zyklisch das Hochdruck-Bypassventil (37) geöffnet und die im Primärkreislauf befindlichen Stoffrückstände zur Drainage (22) über das geöffnete Ventil (20) hin ausgespült.

Optional wird über die Pumpe (61) und eine entweder zusätzlich angeordnete oder allein betriebene Reinigungskammer (38) der Primärkreislauf gereinigt.

Mit den beschriebenen Verfahren ist sind sowohl Primär- als auch Sekundärkreis der Umkehrosomose und des Verteilerkreises mit unterschiedlichen Verfahren, sowie unabhängig voneinander kostensparend und effizient zu reinigen.

Figur 2a zeigt ein Verfahren bei dem die Reinigung des Permeatsammelrohres 48 getrennt vom Permeatkreislauf 23/24 möglich ist.

Dazu wird die hochreine Flüssigkeit der Membran - Sekundärseite 12 mittels Pumpe 27 über Zirkulationsventil 66 und Permeatsammelrohr Zirkulationsleitung 68 solange über die Reinigungsvorrichtungen 32 und/oder 28 geführt bis ein mikrobiologisch inaktivierendes Resultat erzielt wurde.

Mit diesem Verfahren können wahlweise der Permeatkreislauf 23/24 oder der Permeatsammlerkreis 48/23/68 getrennt oder zusammen gereinigt werden.

Das Bypassventil 67 dient dem Ableiten fehlerhaften Permeates bei geschlossenem Permeatring-Absperrventil 35.

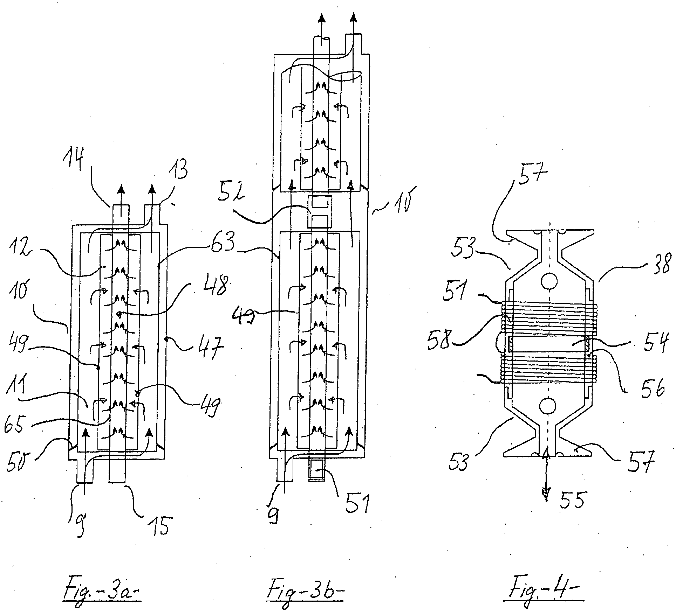

Figur 3a zeigt schematisch ein Modul (10) mit eingesetztem Membranmodul (63). Die Flüssigkeit wird über den Mischwasseranschluss (9) zum Membranprimärraum (11) geführt und gelangt über die Filtermembrane (49) zum Membransekundärraum (12). Das Permeatsammelrohr (48) ist so perforiert (65), dass die über die spiralförmig angeordneten Membrantaschen der Filtermembran (49) zugeführte Flüssigkeit im Membranprimärraum (11) in das Permeat-sammelrohr eintreten kann. Von dort gelangt es über die Anschlüsse (14/15) in den hochreinen Verteilerkreis. Das Konzentrat wird über den Anschluss (13) weitergeleitet.

Figur 3b zeigt dazu im Vergleich eine dem Stand der Technik gemäße 3-Pol-Membran. Auch hier wird die Flüssigkeit über den Anschluss (9) zugeführt. Der Durchtritt über die Filtermembran (49) erfolgt wie bereits unter 3a beschrieben. Schematisch dargestellt sind hier die oft angewandte Technik der Serienschaltung mittels Permeatsammelrohrverbinder (52) und der Verschluss der Permeatseite (51). Die Reinigung des Permeatsammelrohrs kann ausschließlich transmembran erfolgen.

Figur 4 zeigt den Aufbau einer Reinigungszelle (38) mit 3 Elektroden, wobei die mittlere Elektrode (54) räumlich und elektrisch von den beiden Außenelektroden (53) isoliert ist. Die Flüssigkeit kann dabei bidirektional über den Strömungskanal (55) in die Zelle eingeleitet werden. Durch die große Flächenverteilung der Außenelektrode (53) wird eine gleichmäßige Potentialverteilung im Innenelektrodenraum erreicht. Das Isolierstück (56) dient als Einbauraum für die mittlere Elektrode (54). Die becherförmigen Außenelektroden (53) sind mit unterschiedlichen Konnektoren (57) wie z.B. Clampanschluss, Stecknippel-anschluss oder Schlauchanschluss auszustatten.
Die mittlere Elektrode (54) ist als ringförmiger Elektrodenkörper im Isolierstück (56) eingebracht.

Je nach Anwendung ist das Material der Außenelektroden (53) Edelstahl, Titan, Titanmischoxid oder gesinterter Kohle.

Die mittlere Elektrode (54) besteht aus einem oxidationsstabilen Material wie z.B. leitfähigem Kohlenstoff, Titanmischoxid, einem keramischen Gemisch aus Metalloxiden, Titanoxid oder Kobalt.

Durch Materialauswahl und elektrischer Anschlussart ist es möglich die Reinigungskammer (38) als Elektrolysezelle oder als elektromagnetische Zelle oder als Zelle mit Elektrodenanschlüssen für Strom und Spannung - auch kapazitiv - zu betreiben.

Dabei wird vorzugsweise ein Pol der elektrischen Versorgung an die gebrückten Außenelektroden (53) - und der andere Pol an die mittlere Elektrode (54) angelegt.

Bei Betrieb der Reinigungskammer (38) als Elektrolysezelle sind die beiden Außenelektroden (53) die Kathoden, die mittlere Elektrode (54) die Anode.

Diese Elektrolysezelle dient der Herstellung von Sauerstoffradikalen für die Inaktivierung der Mikroorganismen oder auch zur Reduzierung der Kalkablagerung.

Figur 4 zeigt den Aufbau einer kombinierten Reinigungskammer (38) mit 3 Elektroden und einer Spulenwicklung (51).

Hierbei wird die Entkalkung über die Kraftlinien des von der Spule erzeugten Magnetfeldes in der Flüssigkeit durchgeführt.

Die Verwendung von teflongekapselten Ringmagneten in der Flüssigkeit oder Ringmagneten außerhalb des Isolierstückes (56) anstelle der Spulenwicklung (51) ist möglich.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar.

| | |
|---|---|
| 1. | Zuführende Leitung |
| 2. | Messung Eingangsleitfähigkeit |
| 3. | Messung Zufluss |
| 4. | Wassereingangsventil |
| 5. | Vorlaufbehälter |
| 6. | Niveauregelung |
| 7. | Pumpe |
| 8. | RS Ventil |
| 9. | Mischwasserzufluss |
| 10. | Filtermodul |
| 11. | Membranprimärraum |
| 12. | Membransekundärraum |
| 13. | Konzentratabflussle(leitung) |
| 14. | Permeatzuführung |
| 15. | Permeatzirkulations- u. Bypassleitung |
| 16. | Hochdruckrossel |
| 17. | Druckhalteventil |
| 18. | Konzentratüberströmung |
| 19. | Konzentratabflussleitung |
| 20. | Konzentratabflussventil |
| 21. | Konzentratflussmesser |
| 22. | Drainage |
| 23. | Permeatzuführleitunq (können auch in den Verbraucher hineingeführt werden) |
| 24. | Permeatrückführleitung(können auch in den Verbraucher hineingeführt werden) |
| 25. | Konnektionsstelle Verbraucher |
| 26. | Druckhalteventil Permeatrückführung |
| 27. | Zirkulationspumpe |
| 28. | Ozonzelle |
| 29. | Permeatring - Ausströmventil |
| 30. | UV Lampe |
| 31. | Temperatursensor |
| 32. | Heizung |
| 33. | Temperatursensor |
| 34. | Leitfähigkeitszelle |
| 35. | Permeatringabsperrventil |
| 36. | Permeatringsicherheitsventil |
| 37. | Hochdruckdrossel Bypassventil |
| 38. | Reinigungskammer |
| 39. | Heizung Sekundärseite |
| 40. | Zentrifugalkammer |
| 41. | Permeatring - Überströmventil |
| 42. | Schnittstellenleitung |
| 43. | Verbraucher Eingangsventil |
| 44. | Verbraucher |
| 45. | Schnittstellenleitung - Spülventil |
| 46. | Drossel |
| 47. | Druckrohr |
| 48. | Permeatsammelrohr |
| 49. | Filtermembrane |
| 50. | Membrandichtung |
| 51. | Stopfen Permeatsammelrohr |
| 52. | Verbinder Permeatsammelrohr |
| 53. | Außenelektroden |
| 54. | Mittlere Elektrode |
| 55. | Zugeführte Flüssigkeit |
| 56. | Isolierstück |
| 57. | Konnenktoren |
| 58. | Spulenwicklung |
| 59. | RS Ventil |
| 60. | RO-Anlage |
| 61. | Zirkulationspumpe |
| 62. | RS Ventil |
| 63. | Membranmodul |
| 64. | Reinigungssensor |
| 65. | Perforierung Permeatssammelrohr |
| 66. | Zirkulationsventil |
| 67. | Bypassventil |
| 68. | Permeatsammlerzirkulationsleitung |

## Patentansprüche

1. RO-(Umkehrosmose)Anlage, die mit wenigstens einem Dialysegerät koppelbar ist, um dieses mit hochreinem Permeat zu versorgen, mit einer Rohwasserzuflussleitung (1), die Rohwasser durch ein Ventil (4) einem Vorlaufbehälter (5) mit einer Füllstandsregelung zuführt, von dem eine Leitung (9) über ein Pumpe (7) zu einem Filtermodul (10) führt, dessen Primärraum (11) durch eine semipermeable Membran (49) von einem Sekundärraum (12) getrennt ist, der mit einem Verteilungssystem für das Permeat verbunden ist, das eine Permeat-Zulaufleitung (23) mit wenigstens einer Konnektion (25), an die das wenigstens eine Dialysegerät ankoppelbar ist, und eine Permeat-Rücklaufleitung (24) aufweist, in die ein Permeat-Ausströmventil (29) eingesetzt ist, durch das das Permeat in den Vorlaufbehälter (5) ausströmen kann, wobei das Permeat-Verteilungssystem wenigstens eine Einrichtung (32, 28) zur Reinigung und/oder Desinfektion und eine Zirkulationspumpe (27) aufweist, die in einem Zirkulationskreis angeordnet sind, wobei ferner von dem Primärraum (11) des Filtermoduls (10) eine Konzentratabflussleitung zu dem Vorlaufbehälter (5) führt, **dadurch gekennzeichnet, dass** der Sekundärraum (12) durch ein innerhalb von spiralförmig angeordneten Membrantaschen angeordnetes, perforiertes Permeatsammelrohr (48) gebildet ist, das über Anschlüsse an beiden Enden mit dem Zirkulationskreis verbunden ist, indem es mit seinem anderen Ende mit der Permeat-Rücklaufleitung (24) so verbunden ist, dass der Zirkulationskreis gebildet ist, in dem das Permeat zirkulieren kann.

2. RO-(Umkehrosmose)Anlage nach Anspruch 1,
**dadurch gekennzeichnet,**
**dass** der Zirkulationskreis (68) von der Permeat-Zulaufleitung (23) abzweigt und einen Abschnitt davon einschließt.

3. RO-(Umkehrosmose)Anlage nach Anspruch 1 bis 2,
**dadurch gekennzeichnet,**
**dass** in das Permeat-Verteilungssystem ein Rückschlagventil (59) eingesetzt ist, das so wirkt, dass das Permeat von dem Filtermodul (10) aus durch die Permeat-Zulaufleitung (23), nicht jedoch durch die Permeat-Rücklaufleitung (24) strömen kann.

4. RO-(Umkehrosmose)Anlage nach Anspruch 1 bis 3,
**dadurch gekennzeichnet,**
**dass** das Rückschlagventil (59) in der Permeat-Rücklaufleitung (24) dem anderen Ende des Permeatsammelrohres (48) benachbart angeordnet ist.

5. RO-(Umkehrosmose)Anlage nach einem der Ansprüche 1 bis 4,
**dadurch gekennzeichnet,**
**dass** die wenigstens eine Einrichtung zur Reinigung/Desinfektion eine Heizung (32) und eine Temperaturmesseinrichtung (33) aufweist.

6. RO-(Umkehrosmose)Anlage nach einem der Ansprüche 1 bis 5,
**dadurch gekennzeichnet,**
**dass** die wenigstens eine Einrichtung zur Reinigung/Desinfektion wenigstens eine Ozonzelle (28) aufweist.

7. RO-(Umkehrosmose)Anlage nach einem der Ansprüche 1 bis 6,
**dadurch gekennzeichnet,**
**dass** in Strömungsrichtung vor dem wenigstens einen Verbraucher in die Permeat-Zulaufleitung (23) ein Absperrventil (35) eingesetzt ist und dass vor dem Absperrventil (35) eine Bypassleitung in die Permeat-Rücklaufleitung (24) einmündet.

8. RO-(Umkehrosmose)Anlage nach Anspruch 7,
**dadurch gekennzeichnet,**
**dass** zwischen der Einmündung der Bypassleitung und dem wenigstens einem Verbraucher ein den Rücklauf des Permeats zu dem wenigstens einen Verbraucher sperrendes Druckhalteventil (26) in die Permeat-Rücklaufleitung (24) eingesetzt ist.

9. RO-(Umkehrosmose)Anlage nach einem der Ansprüche 7 oder 8,
**dadurch gekennzeichnet,**
**dass** in die Bypassleitung ein den Rücklauf sperrendes Sicherheitsventil (36) eingesetzt ist.

10. RO-(Umkehrosmose)Anlage nach einem der Ansprüche 1 bis 9, wobei die Rohwasserzuflussleitung (9) über eine Rohwasser-Bypassleitung mit der Konzentratabflussleitung (13) verbunden ist,
**dadurch gekennzeichnet,**
**dass** in der Rohwasser-Bypassleitung eine Zirkulationspumpe (61) und eine Reinigungskammer (38) für das Rohwasser eingeschaltet sind.

11. RO-(Umkehrosmose)Anlage nach Anspruch 10,
**dadurch gekennzeichnet,**
**dass** die Reinigungskammer (38) mit vorzugsweise drei Elektroden versehen ist und zur Herstellung von Sauerstoffradikalen für die Inaktivierung von Mikroorganismen und zur Reduzierung von Kalkablagerungen dient.

12. RO-(Umkehrosmose)Anlage nach einem der Ansprüche 1 bis 11,
wobei die Konzentratabflussleitung mit einer Drainage in Verbindung steht, **dadurch gekennzeichnet,**
**dass** in Fließrichtung vor der Drainage (22) eine Zentrifugalkammer mit einem den Grad der Verschmutzung erfassenden, vorzugsweise optischen Sensor (64) angeordnet ist.

## Claims

1. An RO (reverse osmosis) installation, which may be coupled to at least one dialysis device in order to supply it with ultrapure permeate, including a raw water supply conduit (1), which supplies raw water through a valve (4) to a feed tank (5) with a level controller, from which a conduit (9) leads via a pump (7) to a filter module (10), whose primary space (11) is divided by a semipermeable membrane (49) from a secondary space (12), which is connected to a distribution system for the permeate, which includes a permeate supply conduit (23) with at least one connection (25), to which the at least one dialysis device may be coupled, and a permeate return conduit (24), into which a permeate outlet valve (29) is inserted, through which the permeate can flow out into the feed tank (5), wherein the permeate distribution system includes at least one device (32, 28) for cleaning and/or disinfection and a circulation pump (27), which are arranged in a circulation circuit, wherein further a concentrate discharge conduit leads from the primary space (11) of the filter module (10) to the feed tank (5), **characterised in that** the secondary space (12) is constituted by a perforated permeate collection pipe (48), which is arranged within spirally arranged membrane pockets and is connected via connections at both ends to the circulation circuit, its other end being so connected to the permeate return conduit (24) that the circulation circuit is formed, in which the permeate can circulate.

2. An RO (reverse osmosis) installation as claimed in Claim 1, **characterised in that** the circulation circuit (68) branches off from the permeate supply conduit (23) and includes a section of it.

3. An RO (reverse osmosis) installation as claimed in Claims 1 to 2, **characterised in that** inserted into the permeate distribution system there is a non-return valve (59), which acts so that the permeate can flow from the filter module (10) through the permeate supply conduit (23) but not through the permeate return conduit (24).

4. An RO (reverse osmosis) installation as claimed in Claims 1 to 3, **characterised in that** the non-return valve (59) is arranged in the permeate return conduit (24) adjacent to the other end of the permeate collection pipe (48).

5. An RO (reverse osmosis) installation as claimed in one of Claims 1 to 4, **characterised in that** the at least one device for cleaning/disinfection includes a heater (32) and a temperature measuring device (33).

6. An RO (reverse osmosis) installation as claimed in one of Claims 1 to 5, **characterised in that** the at least one device for cleaning/disinfection includes at least one ozone cell (28).

7. An RO (reverse osmosis) installation as claimed in one of Claims 1 to 6, **characterised in that** a shut-off valve (35) is inserted into the permeate supply conduit (23) upstream of the at least one consumer and that a bypass conduit communicates with the permeate return conduit (24) upstream of the shut-off valve (35).

8. An RO (reverse osmosis) installation as claimed in Claim 7, **characterised in that** inserted into the permeate return conduit (24) between the communication of the bypass conduit and the at least one consumer there is a pressure retention valve (26) blocking the return of the permeate to the at least one consumer.

9. An RO (reverse osmosis) installation as claimed in one of Claims 7 or 8, **characterised in that** inserted into the bypass conduit there is a safety valve (36) blocking the return.

10. An RO (reverse osmosis) installation as claimed in one of Claims 1 to 9, wherein the raw water supply conduit (9) is connected to the concentrate outlet conduit (13) by means of a raw water bypass conduit, **characterised in that** connected into the raw water bypass conduit there is a circulation pump (61) and a cleaning chamber (38) for the raw water.

11. An RO (reverse osmosis) installation as claimed in Claim 10, **characterised in that** the cleaning chamber (38) is provided with preferably three electrodes and serves to produce oxygen radicals for the deactivation of microorganisms and to reduce limescale deposits.

12. An RO (reverse osmosis) installation as claimed in one of Claims 1 to 11, wherein the concentrate discharge conduit is connected to a drainage system, **characterised in that** arranged upstream of the drainage system (22) there is a centrifugal chamber, with a preferably optical sensor (64) detecting the degree of contamination.

## Revendications

1. Installation OI (d'osmose inverse), qui peut être couplée à au moins un appareil de dialyse afin d'alimenter en un perméat à haute pureté ledit appareil de dialyse, comprenant une conduite d'arrivée d'eau brute (1), qui amène de l'eau brute par une soupape (4) à un récipient tampon (5) équipé d'un système de régulation de niveau de remplissage, depuis lequel une conduite (9) mène, par une pompe (7), jusqu'à un module filtrant (10), dont l'espace primaire (11) est séparé par une membrane semi-perméable (49) d'un espace secondaire (12), qui est relié à un système de distribution destiné au perméat, lequel présente une conduite d'arrivée de perméat (23) pourvue au moins d'une connexion (25), à laquelle l'appareil de dialyse (44) au moins au nombre de un peut être couplé, et une conduite de reflux de perméat (24), dans laquelle est insérée une soupape d'évacuation par écoulement de perméat (29), laquelle soupape peut évacuer par écoulement le perméat dans le réservoir tampon (5), sachant que le système de distribution de perméat présente au moins un dispositif (32, 28) servant au nettoyage et/ou à la désinfection et une pompe de mise en circulation (27), qui sont disposés dans un circuit de mise en circulation, sachant en outre qu'une conduite d'évacuation de concentré mène, depuis l'espace primaire (11) du module filtrant (10), en direction du récipient tampon (5),
**caractérisée en ce**
**que** l'espace secondaire (12) est formé par un tuyau collecteur de perméat (48) perforé disposé à l'intérieur de poches de membrane disposées de manière à présenter une forme de spirale, lequel tuyau collecteur de perméat est relié par l'intermédiaire de raccords, au niveau des deux extrémités, au circuit de mise en circulation en ce qu'il est relié par son autre extrémité à la conduite de reflux de perméat (24) de telle sorte que le circuit de mise en circulation est formé, dans lequel le perméat peut circuler.

2. Installation OI (d'osmose inverse) selon la revendication 1,
**caractérisée en ce**
**que** le circuit de mise en circulation (68) dévie de la conduite d'arrivée de perméat (23) et en renferme un tronçon.

3. Installation OI (d'osmose inverse) selon la revendication 1 à 2,
**caractérisée en ce**
**qu'**est insérée dans le système de distribution de perméat une soupape de non-retour (59), qui agit de telle manière que le perméat puisse circuler depuis le module filtrant (10) par la conduite d'arrivée de perméat (23), et non pas par la conduite de reflux de perméat (24).

4. Installation OI (d'osmose inverse) selon la revendication 1 à 3,
**caractérisée en ce**
**que** la soupape de non-retour (59) est disposée dans la conduite de reflux de perméat (24) à proximité de l'autre extrémité du tuyau collecteur de perméat (48).

5. Installation OI (d'osmose inverse) selon l'une quelconque des revendications 1 à 4,
**caractérisée en ce**
**que** le dispositif de nettoyage et/ou de désinfection au moins au nombre de un présente un chauffage (32) et un dispositif de mesure de température (33).

6. Installation OI (d'osmose inverse) selon l'une quelconque des revendications 1 à 5,
**caractérisée en ce**
**que** le dispositif de nettoyage/désinfectionau moins au nombre de unun présente au moins une cellule d'ozone (28).

7. Installation OI (d'osmose inverse) selon l'une quelconque des revendications 1 à 6,
**caractérisée en ce**
**qu'**une soupape de blocage (35) est insérée dans le sens d'écoulement, devant le consommateur au moins au nombre d'un, dans la conduite d'arrivée de perméat (23), et
en ce qu'une conduite de dérivation débouche dans la conduite de reflux de perméat (24) avant la soupape de blocage (35).

8. Installation OI (d'osmose inverse) selon la revendication 7,
**caractérisée en ce**
**qu'**une soupape de maintien de pression (26) bloquant le reflux du perméat en direction du consommateur au moins au nombre d'un est insérée dans la conduite de reflux de perméat (24) entre l'embouchure de la conduite de dérivation et le consommateur au moins au nombre de un.

9. Installation OI (d'osmose inverse) selon l'une quelconque des revendications 7 ou 8,
**caractérisée en ce**
**qu'**une soupape de sécurité (36) bloquant le reflux est insérée dans la conduite de dérivation.

10. Installation OI (d'osmose inverse) selon l'une quelconque des revendications 1 à 9, sachant que la conduite d'arrivée d'eau brute (9) est reliée par l'intermédiaire d'une conduite de dérivation d'eau brute à la conduite d'évacuation de concentré (13),
**caractérisée en ce**
**qu'**une pompe de mise en circulation (61) et une chambre de nettoyage (38) pour l'eau brute sont installées dans la conduite de dérivation d'eau brute.

11. Installation OI (d'osmose inverse) selon la revendication 10,
**caractérisée en ce**
**que** la chambre de nettoyage (38) est pourvue de préférence de trois électrodes, et sert à la fabrication de radicaux d'oxygène pour l'inactivation de microorganismes et pour la réduction de dépôts calcaires.

12. Installation OI (d'osmose inverse) selon l'une quelconque des revendications 1 à 11,
sachant que la conduite d'évacuation de concentré est reliée à un système de drainage,
**caractérisée en ce**
**qu'**une chambre pour centrifuger pourvue d'un capteur (64) de préférence optique détectant le niveau d'encrassage est disposée dans le sens d'écoulement avant le système de drainage (22).
